# EUROPEAN PATENT SPECIFICATION

(11) **EP 1 587 573 B1**
(45) Date of publication and mention of the grant of the patent: **25.06.2008**
(21) Application number: 04706990.1
(22) Date of filing: 30.01.2004
(51) Int. Cl.: A61F 2/01, A61B 17/32

(54) **FILTER RETRIEVAL CATHETER SYSTEM, AND METHODS**
FILTER-WIEDERGEWINNUNGS-KATHETERSYSTEM UND VERFAHREN
SYSTEME CATHETER D'EXTRACTION DE FILTRE, ET PROCEDES ASSOCIES

(30) Priority: 31.01.2003 US 444320 P
(43) Date of publication of application: 26.10.2005
(73) Proprietor: Cordis Corporation, Miami Lakes, Florida 33014 (US)
(72) Inventor: FLEMING, James, A., III, Bethlehem, PA 18020 (US)
(74) Representative: Belcher, Simon James
(86) International application number: PCT/US2004/002673
(87) International publication number: WO 2004/069290

(56) References cited:
- WO-A-01/56484
- WO-A1-00/16846
- WO-A2-02/11626
- US-A- 5 895 399
- US-B1- 6 245 087

## Description

### BACKGROUND AND SUMMARY OF THE INVENTION

### 1. Technical Background:

The present invention generally relates to a catheter system to retrieve a filter which has been previously implanted in a body passage for medical treatment of a patient.

### 2. Discussion:

Many different types of filters may be implanted in various body passages for medical treatment of a patient. The present invention will be described by using the specific example of a vascular filter for implantation in a blood vessel, and an example of one particular type of filter retrieval system for retrieving a vascular filter. However, it should be understood that the present invention is not limited to vascular applications, but rather relates to implantable filters for use in any medically suitable type of body passage, for example, the vascular system, biliary system or pulmonary system, esophagus, etc.

Implantable filters may be of various types and designs, including for example permanent or retrievable; they may be made of various types of materials, such as metals and/or polymers; and they may have any of numerous designs. Some implantable filters are resilient, having some degree of "spring" characteristics. This resilience may be present for example along a longitudinal axis of the filter, and/or for example may allow resilient bending along one or more axes of the filter, and/or for example may allow resilient compression of the filter. This latter type of "compressible" filter may be delivered to a desired site for treatment through a catheter system having a passage or lumen containing the filter. When the filter is released from the catheter, it thus tends to resilient expand to a larger size suitable for the size of the body passage at the desired site for treatment.

Permanent filters are not intended for removal, and retrievable filters are intended to be removable for a period of time after being implanted. In other words, a physician has a period of time in which to choose whether to remove a retrievable filter or not. In the specific case of a vascular filter, it may be desirable to have the option to retrieve a filter, for example a filter that has been placed in the inferior vena cava. It may also be desirable to retrieve such vascular filters with a catheter-based retrieval system, from an access point into an artery, for example. Such a catheter-based retrieval system enables the physician to retrieve the filter percutaneously, without performing surgery. In some situations, the desired access point may be into the jugular artery or the femoral artery.

Regarding the filter itself, it may be desirable to provide a medical implantable filter with features(s) for resisting movement of the filter within the body passageway. These features for resisting movement of the filter may be for example one or more resilient anchors, hooks, or barbs, etc. Such movement resistors may be one-directional, resisting displacement in one direction and allowing for removal of the filter in the other direction.

In the example of a vascular filter, the filter may be designed to resist movement from the desired site for treatment in a cranial direction, defined as being opposite a caudal direction. These types of filters are often resilient, and can be radially compressed into a smaller size, and may be removed by a catheter system that is inserted into the femoral artery, has a hook or loop member that passes through a passage or lumen through the catheter which catches the filter, and pulls the filter into the catheter lumen in the caudal direction. The catheter system and captured filter may then be removed from the patient.

It may also be desirable to remove a filter in the cranial direction, by introducing a catheter retrieval system through the jugular artery or brachial artery, and approaching the filter from the cranial direction. However, this approach may not be desirable, if the particular filter is designed to resist movement in the cranial direction.

The retrieval system of the present invention is capable of retrieving a filter when approaching from either direction. In other words, the present catheter-based retrieval system can be inserted through the femoral artery and approach the filter from the caudal direction to retrieve the filter, or through the jugular or brachial artery and approach the filter from the cranial direction, to retrieve the filter. This versatility is provided for approaching the filter from either direction, but enables the movement of the filter during retrieval to be in either direction. Accordingly, the filter can be pulled in the direction of movement that the filter does not resist, regardless of the direction from which the catheter-based retrieval system approaches the filter.

One example of a retrieval catheter system according to the present invention thus allows a filter to be designed to resist movement in the cranial direction, while still permitting retrieval by approaching the filter with a catheter-based retrieval system from either the cranial direction or the caudal direction.

In operation of a vascular filter retrieval system, the system may provide for "pulling" the filter a slight distance in the caudal direction to retract the filter anchors or barbs from engagement with the vessel, before "capturing" the filter within the catheter system, and then retrieving the catheter system and captured filter from whichever access point was selected, and thus in whichever direction is desired. This flexibility of approach may provide greater therapeutic options for treatment of patients.

WO-A-01/56484 discusses a foreign body retrieval device. It includes a catheter having an opening system. A distal end of a wire is attached to the catheter near the opening system and a portion of the wire is oriented external of the catheter. Manipulation of a proximal end of the wire causes a portion of the wire to form a loop external of the catheter that can be used to capture a foreign body.

Accordingly, a general possible object of the present invention is to provide filter retrieval systems for retrieving a previously implanted medical filter, and methods for using the retrieval systems.

Another possible object of this invention is to provide an improved filter retrieval system capable of retrieving a filter in either a proximal or a distal direction, as may be preferred by a physician.

According to the present invention, there is provided a filter retrieval system as defined in appended claim 1.

These and other possible objects, features and advantages of the present invention will be apparent from and clearly understood through a consideration of the following detailed description of the preferred embodiment.

### BRIEF DESCRIPTION OF THE DRAWINGS

In the course of this description of preferred embodiments, reference will be made to the attached drawings, wherein:

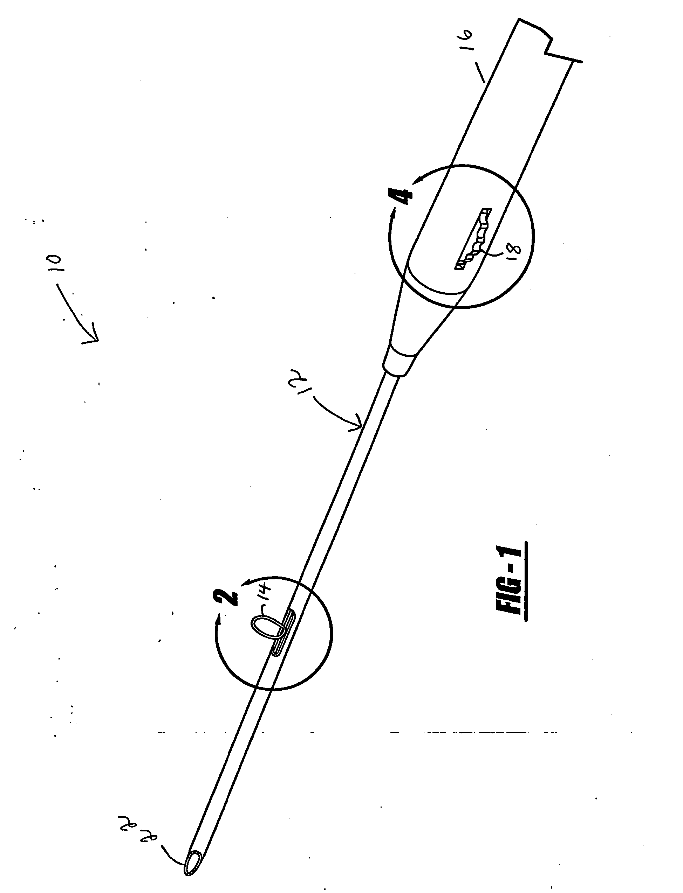

Figure 1 is a perspective view of a catheter-based filter retrieval system, arranged according to the principles of the present invention;

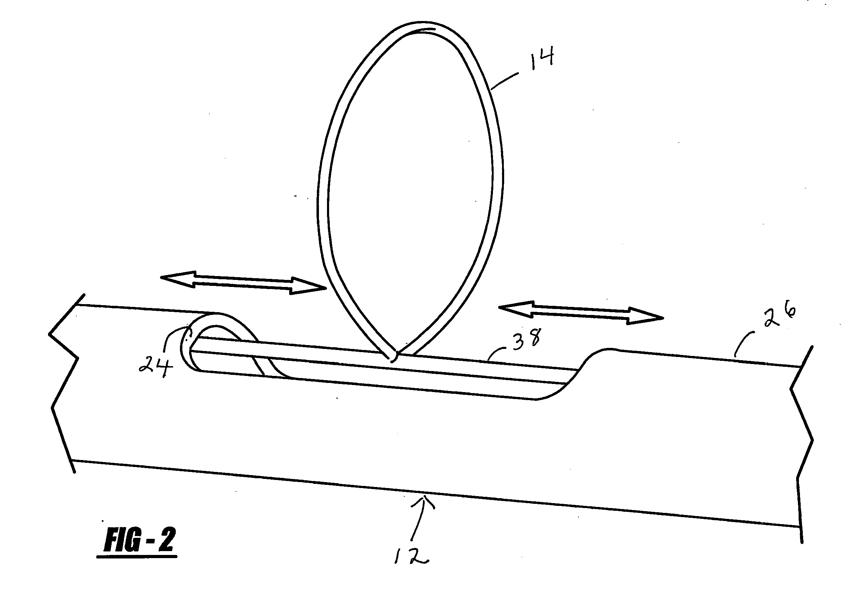

Figure 2 is a partial perspective view of a portion of the filter retrieval system of Figure 1, including a shaft opening and snare loop;

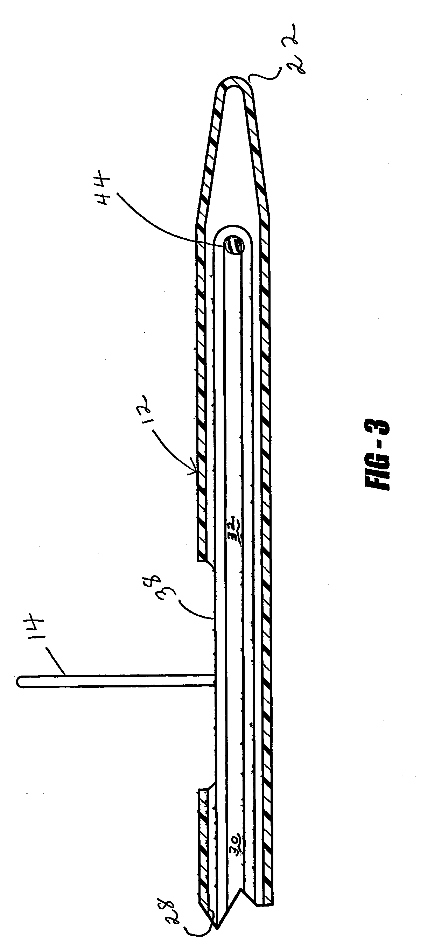

Figure 3 is a partial cross-section view of the distal end of the filter retrieval system of Figure 1;

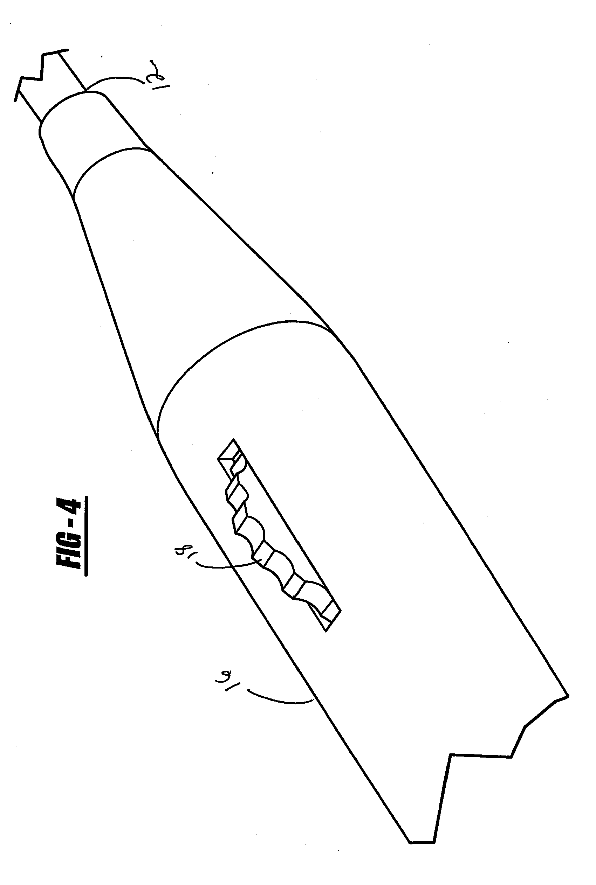

Figure 4 is a partial perspective view of some components of the filter retrieval system of Figure 1, including a handle and actuator wheel;

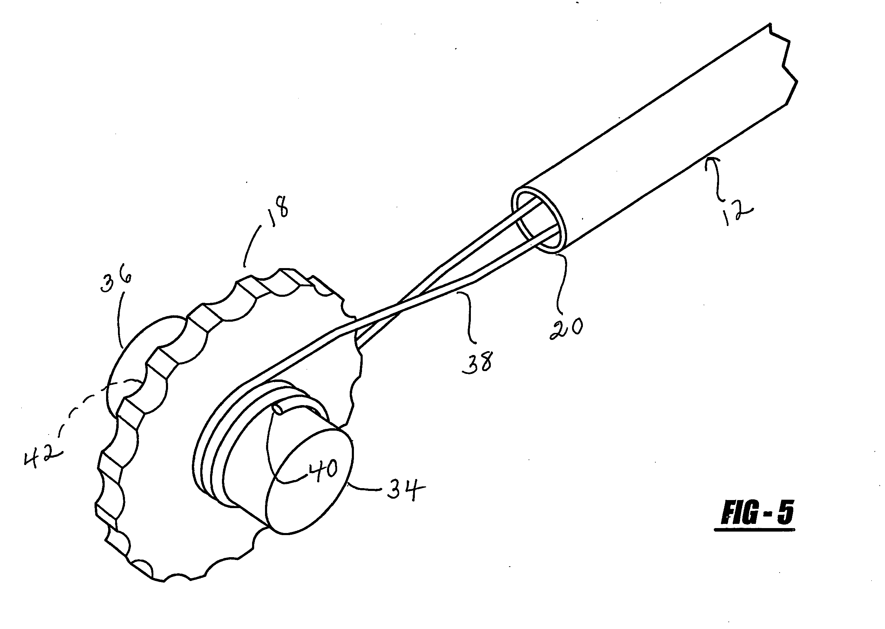

Figure 5 is a partial perspective view of some components of the filter retrieval system of Figure 1, including an actuator wheel, wire and shaft tube;
Figures 6A-6I illustrate a first method of using the system of Figure 1 to retrieve a previously implanted filter in a proximal direction; and
Figures 7A-7J illustrate a second method of using the system of Figure 1 to retrieve a previous implanted filter in a distal direction.

### DESCRIPTION OF THE PREFERRED EMBODIMENTS

The following description of the preferred embodiments of the present invention is merely illustrative in nature, and as such it does not limit in any way the present invention, its application, or uses. Numerous modifications may be made by those skilled in the art without departing from the true spirit and scope of the invention.

One possible arrangement of a filter retrieval system of the present invention is shown in the drawings. Filter retrieval system 10 includes a shaft 12, a retrieval element 14, and a handle 16 with an actuator 18.

The shaft 12 may be catheter-based, and has a proximal and a distal end 20 and 22. The shaft 12 has an opening 24 in its sidewall 26 positioned a distance from the shaft distal end 22. In addition, the shaft 12 may define a lumen or passage 28 from its proximal end 20 to its distal end 22, or at least near its distal end 22. The shaft preferably has an optimum selection of characteristics, including flexibility, column strength or pushability, torque transmission, steerability, etc.

A first and second internal space 30 and 32 are defined inside the shaft 12, located immediately proximal and distal from the side opening 24. The opening 24 or sidehole or slot is preferably spaced from the distal end 22 of the catheter shaft 12 a distance longer or slightly longer than the length of the filter to be retrieved, so that the first and second internal spaces 30 and 32 are sufficiently large to hold the filter inside one of them after the filter has been captured.

The handle 16 is affixed to the shaft 12 near the proximal end 20 of the shaft 12, has an interface for an actuator 18, and provides a comfortable way to manipulate, steer, and direct the retrieval system 10. The actuator 18 is adapted to manipulate the retrieval element 14 by selectively moving the retrieval element 14 in proximal and/or distal directions. The particular actuator 18 shown in the drawings is a wheel having an axle 34 and 36 extending from each side of the wheel. The wheel may have finger ridges as shown.

The actuator 18 should be coupled with the retrieval element 14, so that moving forces applied to the actuator 18 will be transmitted to the retrieval element 14, and move it in a corresponding fashion. The particular construction for transmitting such forces shown in the drawings is a main wire loop 38, extending from a first and second end 40 and 42 of the main wire loop 38, wound around each of the axles 34 and 36 in opposing directions, through a lumen 28 defined by the shaft 12 to a position near the distal end 22 of the shaft 12, where the main wire loop 38 has a 180-degree bend. A pin 44, or pulley or bent tube or 180-degree lumen or any other suitable guide is provided, causing the main wire loop 38 to move around it.

The main wire loop 38 is affixed to the retrieval element 14, so that rotation of the wheel 18 in one direction causes the retrieval element 14 to move in the proximal direction, while rotation of the wheel 18 in the opposite direction causes the retrieval element 14 to move in the distal direction.

The handle 16 and actuator 18 may of course have any preferred design or shape suitable for accomplishing their desired functions. For example, the handle may have a more ergonomic shape, or the actuator may be shaped like a lever, or a slider, etc. The retrieval element may have any shape suitable for grasping, snaring, snagging or catching a corresponding retrieval feature on whatever type of filter is to be retrieved.

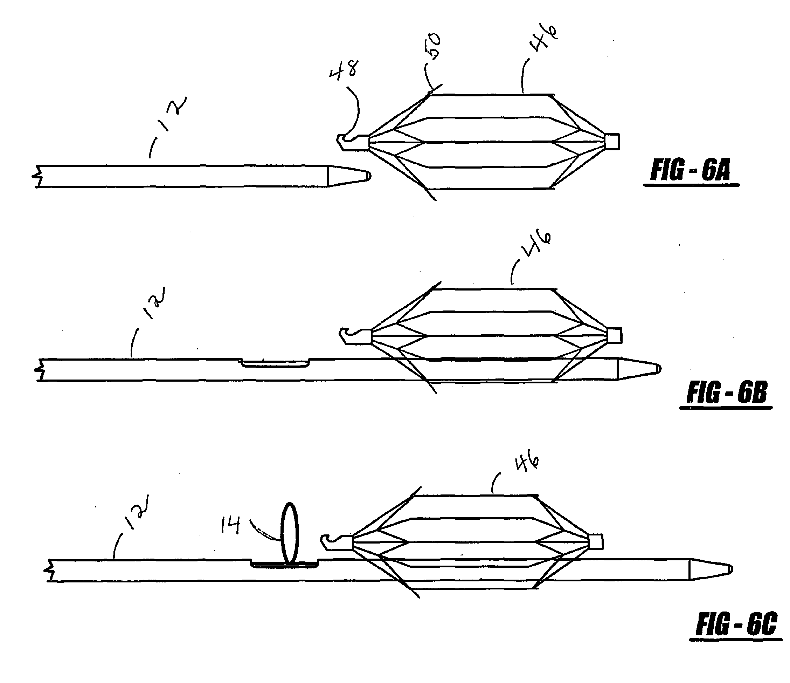

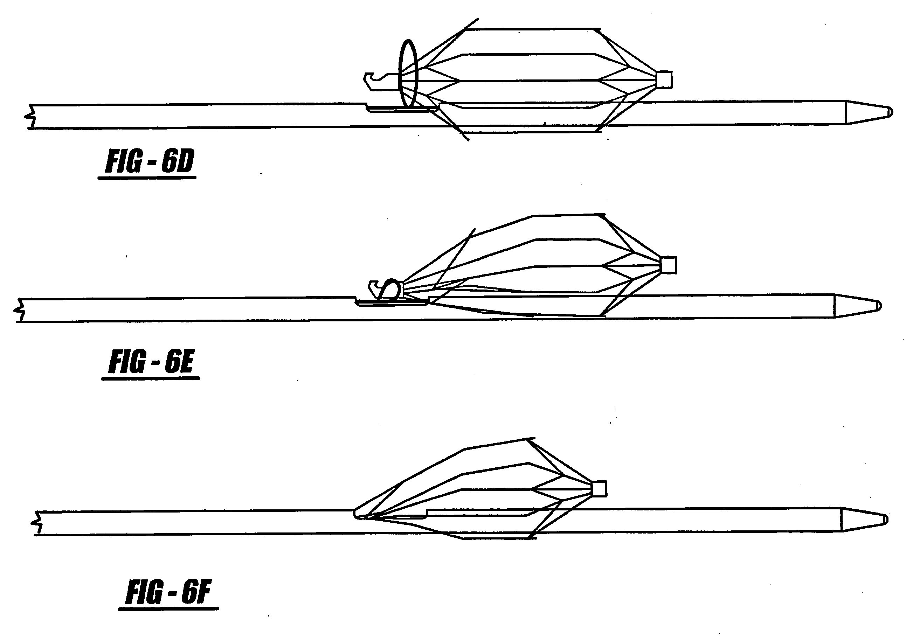

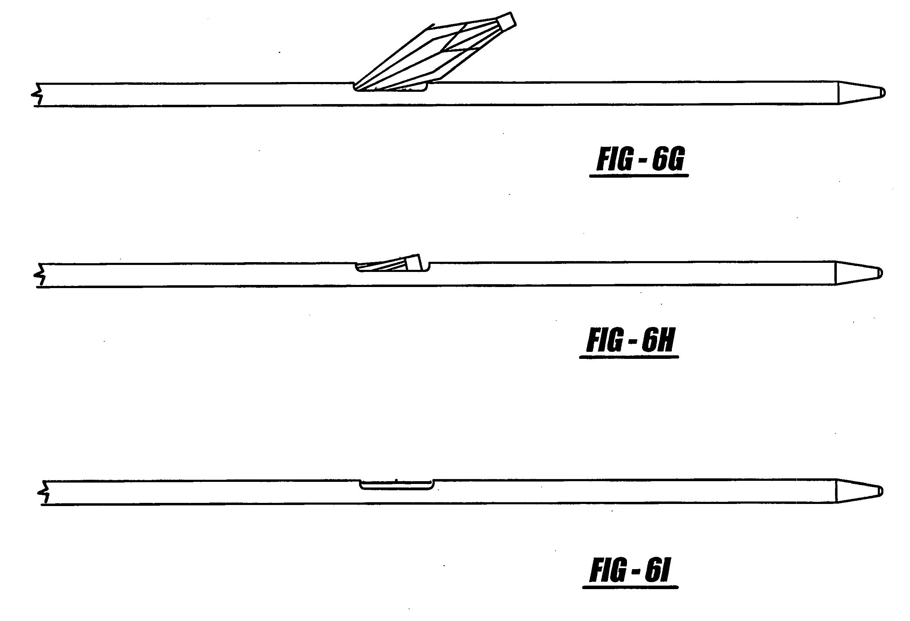

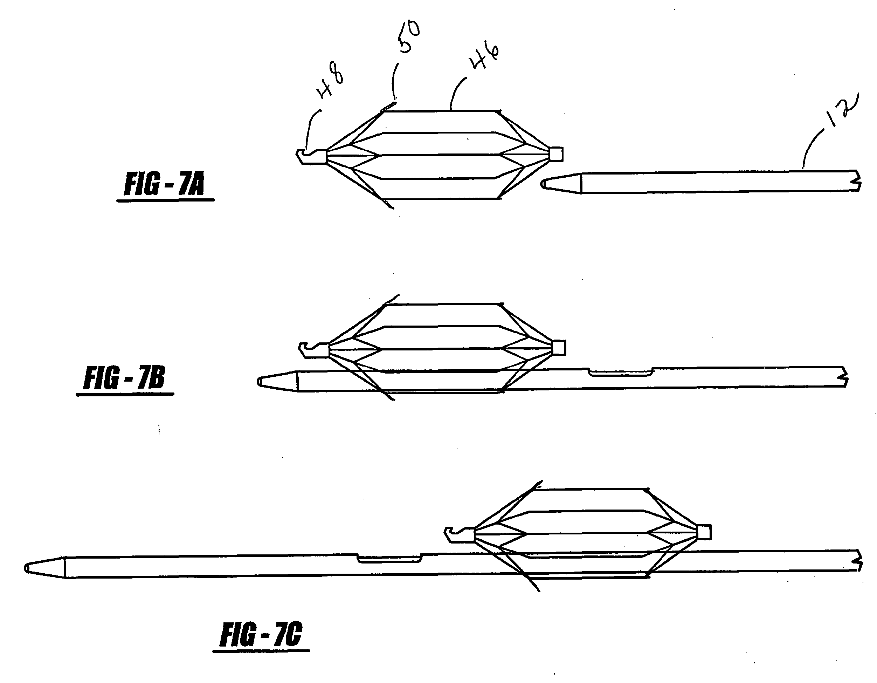

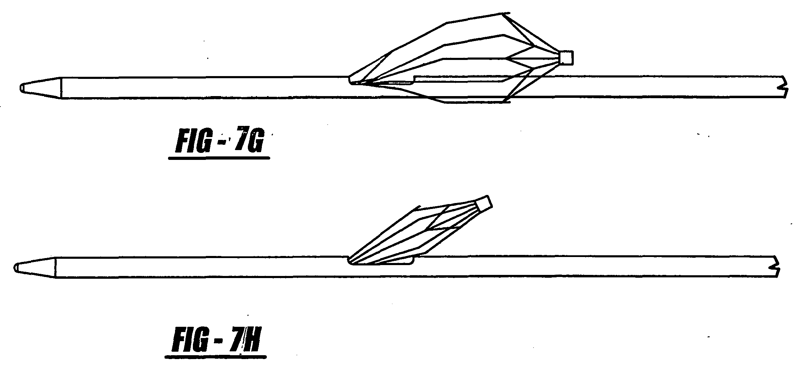

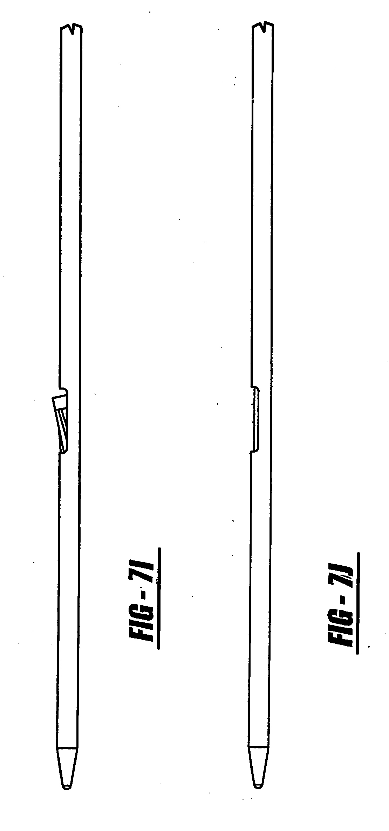

The filter shown in Figures 6A-6I and 7A-7J is one example of numerous filter designs that the filter retrieval systems of the present invention can retrieve. The particular filter 46 depicted in the drawings has a retrieval feature 48 in the form of a hook at one end, and a series of anchors or barbs 50 for resisting movement of the filter 46 within a body passage (which is not shown for clarity purposes).

In operation generally, the retrieval system may inserted into a body passage such as an artery through a percutaneous access point. For example, the access point may be into the femoral, jugular, or brachial arteries. The retrieval system is advanced until the side opening in the shaft is positioned near a desired point on a previously implanted filter. In an initial configuration, the retrieval element is inside the shaft. When the system is suitably positioned, the physician may manipulate the actuator, so that the retrieval element is moved to protrude from the shaft opening. As the actuator is further operated, the retrieval element can grasp or hook or otherwise engage a portion of the filter, and then pull the filter in through the opening into one of the internal spaces of the catheter shaft. The captured filter and the retrieval system may then be removed from the patient. The retrieval system may be capable of retrieving a filter in either a proximal or a distal direction as desired.

In particular, when the catheter system is advanced to position the catheter sidehole and snare near a corresponding retrieval element on the filter, the snare is hooked or attached to the filter retrieval element by manipulating the catheter and spool. Once the snare grabs the filter retrieval element, which may be a hook, loop, catch, barb, etc., the snare and filter may be pulled in through the catheter sidehole in either a proximal or a distal direction depending upon the orientation of the catheter to the filter. The catheter and the captured filter may then be removed from the patient.

To retrieve a filter in the proximal direction, as shown in Figures 6A-6B, the distal end 22 of the shaft 12 is advanced to a position near the filter 46 until the sidehole 24 is aligned with the filter retrieval feature 48, which may be for example one or more hooks, barbs, loops, etc.

The wheel 18 is rotated slightly to move the main wire loop 38 inside the catheter shaft 12 to expose the retrieval element or snare 14, shown in Figure 6C.

The catheter shaft is pushed or advanced toward the filter so that the snare 14 is advanced over the filter retrieval feature 48 or attachment point, shown in Figure 6D.

The wheel 18 is again rotated to pull the main wire loop 38 inside the catheter shaft 12 in the proximal direction, to draw the filter 46 into the catheter shaft 12 until it is inside the proximal internal space 30, shown in Figures 6E-6I. The retrieval system and captured filter are then removed from the body.

Likewise, to retrieve a filter in the distal direction, as shown in Figures 7A-7C, the distal end 22 of the shaft 12 is advanced to a position past the filter 46, until the sidehole 24 is aligned with the filter retrieval feature 48, which may be for example one or more hooks, barbs, loops, etc.

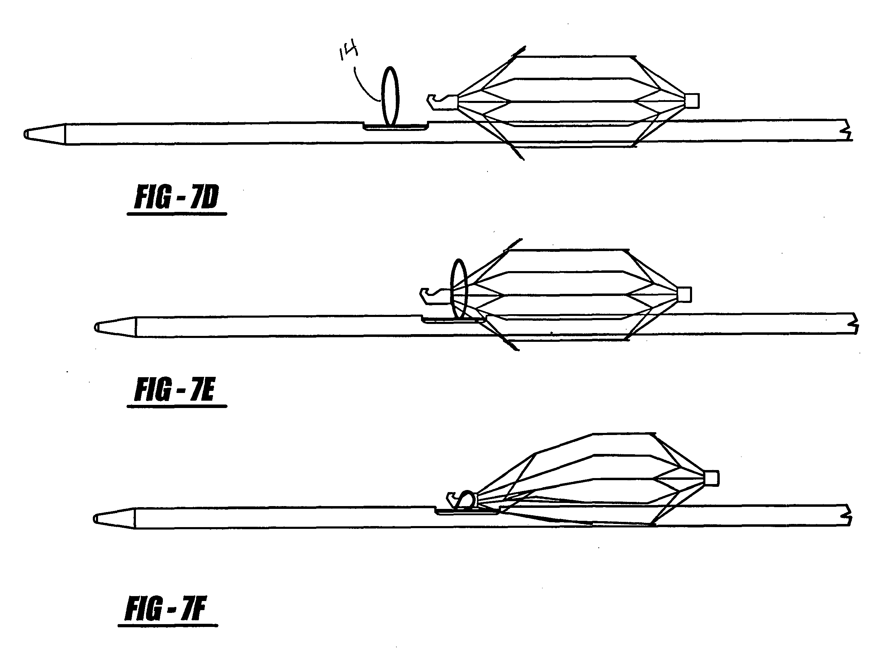

The wheel 18 is rotated slightly to move the main wire loop 38 inside the catheter shaft 12 to expose the retrieval element or snare 14, shown in Figure 7D.

The catheter shaft is pulled or withdrawn in the proximal direction so the snare 14 moves toward the filter, so that the snare 14 surrounds the filter retrieval feature 48 or attachment point, shown in Figure 7E.

The wheel 18 is again rotated to pull the snare 14 in the distal direction, to draw the filter 46 into the catheter shaft 12 until it is inside the distal internal space 32, shown in Figures 7F-7J. The retrieval system and captured filter are then removed from the body.

The components of the retrieval systems of the present invention may be made of any suitable material having the desired performance characteristics, including biocompatibility, strength, flexibility, etc. For example, in the particular retrieval system shown in the drawings, the handle and wheel may be made of metals, polymers, or even ceramics, while the shaft may be made of any suitable polymer, possibly in combination with a metal component such as a hypotube, and the retrieval element and main wire loop are preferably made of metal or a filament material.

The total length of the catheter may be selected as any appropriate length, which may be approximately 50-100 centimeters, to allow access to the desired sites for treatment from various insertion points.

Numerous arrangements of and modifications to the present invention will be readily apparent to those skilled in the art. For example, the portion of the main wire loop inside of the catheter shaft that does not have the snare attached to it may be contained inside a separate lumen of the catheter. Also, a separate guide wire lumen and a distal guidewire port may be provided in the catheter shaft to allow the retrieval system to be inserted over a guidewire. Also, the distal end of the catheter shaft may be pre-curved or shaped, and/or may be steerable by means of a separate wire or filament to improve the alignment of the retrieved filter to the opening of the catheter shaft. The actuator may be replaced with any suitable mechanism, including for example a slider. Also, instead of using a spool, two ends of the main wire used to manipulate the snare may simply pass through the handle or hub so that the physician may operate them individually. Also, the edges of the sidehole in the catheter may be flared or shaped to facilitate entry of the filter. Also, the shaft or main wire loop may be provided with radiopaque marker bands, which are visible on a fluoroscope or X-ray video screen.

In addition, the retrieval systems of the present invention may be used to retrieve any type of implantable medical device that has a suitable retrieval feature, and is radially compressible and capturable within the retrieval system.

## Claims

1. A filter retrieval system (10) for retrieving a previously implanted medical filter, comprising:
a flexible shaft (12) defining a longitudinal axis having a proximal (20) and a distal (22) end; the shaft having an opening (24) positioned a distance from the shaft distal end (22);
a handle (16) affixed to the shaft (12) near the shaft proximal end (20);
an actuator (18) coupled with the handle (16); and
a retrieval element (14) coupled with the actuator (18);
such that moving the actuator (18) to a desired position causes the retrieval element (14) to move to a corresponding position;
such that when the actuator (18) is moved to a ready position, the retrieval element (14) protrudes from the shaft opening (24); such that the retrieval element (14) can be maneuvered to engage a portion of a filter; **characterised in that**
the shaft (12) defines a first (30) and second (32) internal space positioned proximally and distally of the opening (24);
the retrieval element (14) is longitudinally movable among a range of positions, including the position of the shaft opening (24) and the positions of the proximal and distal internal spaces;
such that when the retrieval element (14) is in an initial position, the retrieval element (14) is located within one of the first and second internal spaces (30, 32);
such that when the actuator (18) is moved to a ready position and the retrieval element (14) can be maneuvered to engage a portion of the filter, the actuator (18) can subsequently be moved to a first or second capture position which pulls the filter through the opening (24) into a selected one of the first and second internal spaces (30, 32).

2. The filter retrieval system (10) in accordance with claim 1, wherein the longitudinal axis and the shaft (12) follow a curved path.

3. The filter retrieval system (10) in accordance with claim 1, further comprising a guidewire lumen (28) defined by the shaft (12) and a distal guidewire port near the shaft distal end (22), for slidably receiving a guidewire.

4. The filter retrieval system (10) in accordance with claim 1, wherein a distal portion of the shaft (12) has a pre-selected shape.

5. The filter retrieval system (10) in accordance with claim 1, further comprising one or more marker bands visible on a fluoroscope.

6. A filter retrieval system (10) according to claim 1, wherein the retrieval element (14) is a snare, and the filter retrieval system (10) further comprises a main wire loop (38) coupled with the actuator (18), and having the snare; such that moving the actuator (18) to a desired position causes the main wire loop (38) to move the snare to a corresponding position.

## Patentansprüche

1. Filterrückholsystem (10) zum Rückholen eines vorher implantierten medizinischen Filters, das Folgendes umfasst:
einen flexiblen Schaft (12), der eine Längsachse definiert mit einem proximalen Ende (20) und einem distalen Ende (22), wobei der Schaft eine Öffnung (24) aufweist, die sich in einem Abstand von dem distalen Schaftende (22) befindet;
ein Handstück (16), das an dem Schaft (12) nahe dem proximalen Schaftende (20) angebracht ist;
ein Aktuator (18), der mit dem Handstück (16) gekoppelt ist; und
ein Rückholelement (14), das mit dem Aktuator (18) gekoppelt ist;
so dass eine Bewegung des Aktuators (18) in eine gewünschte Position eine Bewegung des Rückholelements (14) in eine entsprechende Position hervorruft;
so dass, wenn der Aktuator (18) in eine Betriebsposition bewegt wird, das Rückholelement (14) aus der Schaftöffnung (24) hervorsteht, so dass das Rückholelement (14) manövriert werden kann, um einen Teil eines Filters zu ergreifen; **dadurch gekennzeichnet, dass**
der Schaft (12) einen ersten (30) und einen zweiten (32) Innenraum definiert, die sich proximal und distal der Öffnung (24) befinden;
das Rückholelement (14) in Längsrichtung über einen Bereich von Positionen beweglich ist, einschließlich der Position in der Schaftöffnung (24) und den Positionen des proximalen und distalen Innenraums;
so dass, wenn sich das Rückholelement (14) sich in einer Anfangsposition befindet, das Rückholelement (14) in dem ersten oder zweiten Innenraum (30, 32) angeordnet ist;
so dass, wenn der Aktuator (18) in eine Betriebsposition bewegt wird und das Rückholelement (14) manövriert werden kann, um einen Teil des Filters zu ergreifen, der Aktuator (18) nachfolgend in eine erste oder zweite Ergreifungsposition bewegt werden kann, welche den Filter durch die Öffnung (24) in einen ausgewählten der ersten und zweiten Innenräume (30, 32) zieht.

2. Filterrückholsystem (10) nach Anspruch 1, bei dem die Längsachse und der Schaft (12) einem gekrümmten Weg folgen.

3. Filterrückholsystem (10) nach Anspruch 1, das weiterhin ein Führungsdrahtlumen (28), das durch den Schaft (12) definiert ist, und einen distalen Führungsdrahtanschluss nahe dem distalen Schaftende (22) zum gleitfähigen Aufnehmen eines Führungsdrahtes aufweist.

4. Filterrückholsystem (10) nach Anspruch 1, in dem der distale Teil des Schafts (12) eine vorgewählte Form aufweist.

5. Filterrückholsystem (10) nach Anspruch 1, das ferner ein oder mehrere Markierungsbänder aufweist, die auf einem Röntgengerät sichtbar sind.

6. Filterrückholsystem (10) nach Anspruch 1, bei dem das Rückholelement (14) eine Schlinge ist und das Filterrückholsystem (10) weiterhin eine Hauptdrahtschleife (38) aufweist, die mit dem Aktuator (18) gekoppelt ist und die Schlinge aufweist, so dass eine Bewegung des Aktuators (18) in eine gewünschte Position bewirkt, dass die Hauptdrahtschleife (38) die Schlinge in eine entsprechende Position bewegt.

## Revendications

1. Système de récupération de filtre (10) pour récupérer un filtre médical implanté précédemment, comprenant:
◆ une tige flexible (12) définissant un axe longitudinal ayant une extrémité proximale (20) et une extrémité distale (22) ; la tige présentant une ouverture (24) positionnée à une distance de l'extrémité distale (22) de la tige ;
◆ une poignée (16) fixée sur la tige (12) au voisinage de l'extrémité proximale (20) de la tige ;
◆ un dispositif d'actionnement (18) couplé à la poignée (16); et
◆ un élément de récupération (14) couplé au dispositif d'actionnement (18);
de sorte que le déplacement du dispositif de d'actionnement (18) dans une position désirée provoque le déplacement de l'élément de récupération (14) dans une position correspondante ;
de sorte que, lorsque le dispositif d'actionnement (18) est déplacé dans une position d'attente, l'élément de récupération (14) fait saillie depuis l'ouverture de la tige (24) ; de sorte que l'élément de récupération (14) peut être manoeuvré pour mettre en prise une portion du filtre ; **caractérisé en ce que**
◆ la tige (12) définit un premier (30) et un second (32) espaces internes positionnés de manière distale et de manière proximale par rapport à l'ouverture (24) ;
◆ l'élément de récupération (14) peut se déplacer longitudinalement parmi une gamme de positions, y compris la position d'ouverture (24) de la tige et les positions des espaces internes proximal et distal ;
de sorte que, lorsque l'élément de récupération (14) est dans une position initiale, l'élément de récupération (14) est situé au sein de l'un du premier et du second espaces internes (30, 32);
de sorte que, lorsque le dispositif de d'actionnement (18) est déplacé dans une position d'attente et que l'élément de récupération (14) peut être manoeuvré pour mettre en prise une portion du filtre, le dispositif d'actionnement (18) peut être déplacé par la suite dans une première ou une seconde position de capture qui tire le filtre à travers l'ouverture (24) dans un espace sélectionné parmi le premier et le second espaces internes (30, 32).

2. Système de récupération de filtre (10) selon la revendication 1, dans lequel l'axe longitudinal et la tige (12) suivent une trajectoire courbe.

3. Système de récupération (10) selon la revendication 1, comprenant en outre une lumière de fil de guidage (28) définie par la tige (12) et un orifice de fil de guidage distal au voisinage de l'extrémité distale de la tige (22), de manière à réceptionner de façon glissante un fil de guidage.

4. Système de récupération de filtre (10) selon la revendication 1, dans lequel une portion distale de la tige (12) a une forme prédéterminée.

5. Système de récupération de filtre (10) selon la revendication 1, comprenant en outre une ou plusieurs bandes de marquage visibles sur un fluoroscope.

6. Système de récupération de filtre (10) selon la revendication 1, dans lequel l'élément de récupération (14) est un noeud coulant et le système de récupération de filtre (10) comprend en outre une boucle de fil principal (38) couplée au dispositif d'actionnement (18) et portant le noeud coulant; de telle sorte que le déplacement du dispositif d'actionnement (18) dans une position désirée provoque le déplacement de la boucle de fil principal (38) pour déplacer le noeud coulant dans une position correspondante.
